# EUROPÄISCHE PATENTSCHRIFT

(11) **EP 2 800 974 B1**
(45) Veröffentlichungstag und Bekanntmachung des Hinweises auf die Patenterteilung: **14.12.2016**
(21) Anmeldenummer: 13705715.4
(22) Anmeldetag: 03.01.2013
(51) Int. Cl.: G01N 33/569, C07K 14/74, G01N 33/566

(54) **VERFAHREN ZUM HERSTELLEN EINES UNTERSUCHUNGSREAGENS UND KIT ZUM ANALYSIEREN EINER T-ZELLEN-FREQUENZ**
METHOD FOR PRODUCING AN EXAMINATION REAGENT AND KIT FOR ANALYSING A T-CELL FREQUENCY
PROCÉDÉ DE PRÉPARATION D'UN RÉACTIF DE RECHERCHE ET KIT D'ANALYSE D'UNE FRÉQUENCE DE LYMPHOCYTES T

(30) Priorität: 04.01.2012 DE 202012100019 U; 08.02.2012 DE 102012101028
(43) Veröffentlichungstag der Anmeldung: 12.11.2014
(73) Patentinhaber: Jacobs University Bremen GmbH, 28759 Bremen (DE)
(72) Erfinder: SPRINGER, Sebastian, Hartmut, 28717 Bremen (DE); SAINI, Sunil, Kumar, Kerala 689121 (IN)
(74) Vertreter: Weidner Stern Jeschke
(86) Internationale Anmeldenummer: PCT/DE2013/100001
(87) Internationale Veröffentlichungsnummer: WO 2013/102458

(56) Entgegenhaltungen:
- EP-A1- 1 683 808
- RODENKO B ET AL: "Generation of peptide-MHC class I complexes through UV-mediated ligand exchange", NATURE PROTOCOLS, NATURE PUBLISHING GROUP, GB, Bd. 1, Nr. 3, 1. Januar 2006 (2006-01-01), Seiten 1120-1131, XP003027415, ISSN: 1750-2799, DOI: 10.1038/NPROT.2006.121 in der Anmeldung erwähnt
- TOEBES M ET AL: "DESIGN AND USE OF CONDITIONAL MHC CLASS I LIGANDS", NATURE MEDICINE, NATURE PUBLISHING GROUP, NEW YORK, NY, US, Bd. 12, Nr. 2, 5. Februar 2006 (2006-02-05), Seiten 246-251, XP007900182, ISSN: 1078-8956, DOI: 10.1038/NM1360
- BORIS RODENKO ET AL: "Class I major histocompatibility complexes loaded by a periodate trigger", JOURNAL OF THE AMERICAN CHEMICAL SOCIETY, ACS PUBLICATIONS, US, Bd. 131, Nr. 34, 1. August 2009 (2009-08-01), Seiten 13205-13213, XP002658373, ISSN: 0002-7863, DOI: 10.1021/JA9037565 [gefunden am 2009-08-05]
- P. V. K. PRAVEEN ET AL: "Tapasin edits peptides on MHC class I molecules by accelerating peptide exchange", EUROPEAN JOURNAL OF IMMUNOLOGY, Bd. 40, Nr. 1, 16. Januar 2010 (2010-01-16), Seiten 214-224, XP55064794, ISSN: 0014-2980, DOI: 10.1002/eji.200939342
- MARTIN ZACHARIAS ET AL: "Conformational Flexibility of the MHC Class I [alpha]1-[alpha]2 Domain in Peptide Bound and Free States: A Molecular Dynamics Simulation Study", BIOPHYSICAL JOURNAL, Bd. 87, Nr. 4, 1. Oktober 2004 (2004-10-01), Seiten 2203-2214, XP55064799, ISSN: 0006-3495, DOI: 10.1529/biophysj.104.044743
- CHEN L ET AL: 'Endolysosomal processing of exogenous antigen into major histocompatibility complex class I-binding peptides' SCANDINAVIAN JOURNAL OF IMMUNOLOGY Bd. 59, Nr. 6, Juni 2004, Seiten 545 - 552, XP008177359 ISSN: 0300-9475
- KUNIYASU ET AL: "Blocking intrahepatic deletion of activated CD8<+> T cells by an altered peptide ligand", CELLULAR IMMUNOLOGY, ACADEMIC PRESS, SAN DIEGO, CA, US, vol. 238, no. 1, 1 November 2005 (2005-11-01), pages 31-37, XP005300508, ISSN: 0008-8749, DOI: 10.1016/J.CELLIMM.2005.12.006

### SEQUENCE LISTING

<110> Jacobs University Bremen gGmbH
<120> Verfahren zum Herstellen eines Untersuchungsreagens und Kit zum Analysieren einer T-Zellen-Frequenz
<130> P00545WO
<150> 202012100019.9
   <151> 2012-01-04
<150> 102012101028.5
   <151> 2012-02-08
<160> 3
<170> PatentIn version 3.5
<210> 1
   <211> 12
   <212> PRT
   <213> Unknown
<220>
   <223> Linker
<400> 1
   Gly Gly Gly Ser Gly Gly Gly Ser Gly Gly Gly Ser
   1               5                   10
<210> 2
   <211> 9
   <212> PRT
   <213> Unknown
<220>
   <223> Helfer-Ligand
<400> 2
   Phe Ala Pro Gly Asn Tyr Pro Ala Leu
   1               5
<210> 3
   <211> 8
   <212> PRT
   <213> Unknown
<220>
   <223> Untersuchungspeptid
<400> 3
   Ser Ile Ile Asn Phe Glu Lys Leu
   1               5

## Beschreibung

Die Erfindung betrifft ein Verfahren zum Herstellen eines Untersuchungsreagens, ein Multimer, ein Vorlösungsuntersuchungsreagens, ein Untersuchungsreagens, eine Analyselösung, ein Kit, ein Verfahren zum Erhalt eines Analyseergebnisses und ein Analyseergebnis.

MHC-Klasse-I-Proteine (MHC-Klasse-I-Moleküle, Major Histocompatibility Complex Class I molecules, Haupt-Histokompatibilitäts-Komplex-Klasse-I-Proteine) sind Transmembranproteine der zellulären Immunantwort, die antigene Peptide aus dem Zellinneren einer antigenpräsentierenden Zelle (APC), wie beispielsweise aus dem Cytosol oder dem Lumen der endocytischen Organellen, binden und an der Zelloberfläche den zytotoxischen T-Zellen (engl. CTL, cytotoxic T lymphocytes, killer T cells, CD8 positive lymphocytes) präsentieren. Dies ist die so genannte Klasse-I-Antigenpräsentation.

Die Bindung eines T-Zell-Rezeptors (TCR) einer CTL an einen Klasse-I-Peptid-Komplex einer APC führt (abhängig vom Ort der Reaktion im Körper, dem Typ der APC (B-Zelle, dendritische Zelle, etc.) und dem Aktivierungszustand der CTL) zur Aktivierung der CTL und/oder zur Induktion des Zelltods (Apoptose) der APC durch die CTL.

Die Spezifität (Reaktionsfähigkeit mit einem bestimmten Klasse-I-Peptid-Komplex) einer CTL liegt darin begründet, dass sie an ihrer Oberfläche nur eine Art TCR (d.h., nur TCRs einer einzelnen eindeutigen Sequenz) trägt. Die Immunantwort ist effektiv, weil solche CTL, die mit Selbst-Peptiden (Peptiden, die die aus körpereigenen Proteinen erzeugt werden) reagieren, im Thymus eliminiert werden.

Aus diesem Grund bedeutet die Erkennung eines antigenen Peptids durch den TCR einer CTL, dass die APC fremde Proteine produziert, die von Viren oder intrazellulären Parasiten (Bakterien, Protozoen) stammen könnten. Auch eine Überproduktion endogener Peptide in malignen entarteten Zellen, zum Beispiel in Tumoren, kann zu solchen Erkennungsreaktionen führen.

Nahezu alle in der Zelle vorhandenen Proteine werden in Peptide zersetzt, die dann im Endoplasmatischen Retikulum (einer membranumgrenzten Organelle im Zellinneren) an MHC-Klasse-I-Proteine binden. Anschließend wird der Komplex aus Peptid und MHC-Klasse-I-Protein (hier auch kurz als Klasse-I-Peptid-Komplex bezeichnet) an die Zelloberfläche transportiert und steht dort für die Erkennung durch CTL zur Verfügung.

Wenn nun aufgrund einer tumorigenen malignen Entartung neuartige oder mutierte Proteine produziert werden oder wenn durch eine virale oder bakterielle Infektion virale oder bakterielle Proteine aus dem genetischen Material des Virus oder Bakteriums produziert werden, werden diese 'neuartigen' Proteine ebenfalls in Peptide zersetzt, die dann im Komplex mit MHC-Klasse-I-Proteinen auf der Zelloberfläche präsentiert werden. Diese 'neuartigen' Peptide sind verschieden von den zelleigenen und lösen eine Erkennung durch die CTL aus.

Die Präsentation durch MHC-Klasse-I-Proteine spielt ebenfalls eine Rolle in allergischen Reaktionen, der Abstoßung von Transplantaten und einer Anzahl von Autoimmunkrankheiten wie multipler Sklerose und Spondyloarthropathien (z.B. Morbus Bechterew oder rheumatoider Arthritis).

Die Untersuchung der Immunantworten, die durch MHC-Klasse-I-Proteine vermittelt werden, erfordert oft den Nachweis derjenigen CTL, die mit einem bestimmten Klasse-I-Peptid-Komplex (Epitop) reagieren. Reaktionen auf ein einziges immunodominantes Epitop machen oft 10-20% der gesamten T-Zell-Population in einem Organismus aus, und die Verfolgung der T-Zell-Frequenz (d.h. des Anteils derjenigen CTL, die mit einem bestimmten Epitop reagieren, an der Gesamtmenge der CTL im Organismus) erlaubt es daher, die Immunantwort (und z.B. Gelingen oder Fehlschlagen einer Therapie) genau zu verfolgen. Aus diesem Grund sind Reagenzien, die die Epitop-Spezifität von CTL identifizieren können, unabdingbar.

Zum Nachweis solcher Epitop-spezifischer CTL bedient man sich bisher rekombinanter MHC-Klasse-I-Proteine, die in Bakterien hergestellt werden und als unlösliche Einschlusskörper (inclusion bodies) vorliegen, die in einer Lösung eines chaotropen Agens zunächst denaturiert werden. Das Chaotrop wird dann in Gegenwart des gewünschten Peptids entfernt, und der Klasse-I-Peptid-Komplex (falls erforderlich) durch Gelfiltrationschromatographie von dem ungefalteten Protein getrennt. Da die niedrige Affinität eines einzelnen Klasse-I-Peptid-Komplexes zu einem einzelnen TCR nicht zu einer festen Bindung führt, verwendet man Multimere von Klasse-I-Peptid-Komplexen, die aufgrund des Aviditätseffektes fest genug an die TCR einer CTL binden, um eine dauerhafte Bindung zu ermöglichen.

Solche Multimere werden z.B. durch Streptravidinvermittelte Tetramerisierung von biotinylierten Klasse-I-Peptid-Komplexen (Klasse-I-Tetramere, class I tetramers), durch Pentamerisierung durch selbst-assemblierende Coiled-Coil-Domänen (Klasse-I-Pentamere, class I pentamers), oder durch Multimerisierung an Dextran (Klasse-I-Dextramere) erreicht. Weitere Methoden zur Oligo- oder Multimerisierung von Klasse-I-Peptid-Komplexen existieren.

Die Klasse-I-Multimere sind im Allgemeinen mit Fluoreszenzfarbstoffen markiert, die ihre mikroskopische oder durchflusszytometrische Detektion ermöglichen. So können epitopspezifische CTL zum Zwecke des Nachweises direkt angefärbt werden.

Andere Anwendungen für rekombinante Klasse-I-Peptid-Komplexe sind:
- **In vitro -** Selektion und Expansion monospezifischer T-Zellen zur Reinfusion bei Krebs und Viruskrankheiten. (Die Selektion kann durch Cytofluorimetrie (Durchflusszytometrie, FACS), durch Magnet-aktivierte Zellsortierung (MACS) oder - für erhöhten Durchsatz - in Mikroarrays erfolgen.)
- **Ex vivo** - Isolierung und Expansion von CTL zur adoptiven Therapie nach allogeneischer Stammzell-Transplantation.
- **Ex vivo** - Entfernung von alloreaktiven T-Zellen nach Transplantation von peripheren Stammzellen. Ebenso interessant ist die Entfernung von autoreaktiven T-Zellen, die Typ-I-Diabetes, Arthritis, und andere Autoimmun-Krankheiten hervorrufen, wie für MHC-Klasse-II-Reagenzien bereits beschrieben. Der Gebrauch von isotopmarkierten Multimeren ist in US. Pat. US 2003/0228258 A1 "Suicide tetramers and uses thereof", David Scheinberg et al., beschrieben.

Die Herstellung rekombinanter Klasse-I-Peptid-Komplexe ist kompliziert, langwierig und teuer. Zum einen sind mehrere tausend MHC-Klasse-I-Allotypen bekannt (von denen allerdings bereits fünf Allele von HLA-A etwa 50% der Welt-Bevölkerung abdecken). Vor allem aber muss für jedes antigene Peptid, das als Epitop untersucht werden soll, ein neues Multimer hergestellt werden, sodass für jeden Patienten oder für jedes Experiment neue Multimere erforderlich sind, die nach Bedarf spezifisch hergestellt werden müssen.

Es wäre einfacher, die Multimere ohne die antigenen Peptide herzustellen und die jeweils erforderlichen antigenen Peptide dann je nach Bedarf zuzugeben, aber das ist bisher nicht möglich, weil die Faltung der MHC-Klasse-I-Proteine ohne antigenes Peptid für die meisten MHC-Klasse-I-Allotypen nicht möglich ist.

In der Vergangenheit ist ein Ausweg beschrieben worden. Dabei wurde Gebrauch von einem speziellen Peptid gemacht, welches durch UV-Bestrahlung oder Periodat-Behandlung zerfällt und dann durch ein zugegebenes Untersuchungspeptid ersetzt werden kann (Publikation: Rodenko et al., Nature Protocols 1 (2006), S. 1120).

Diese Methode ist teuer, kompliziert und funktioniert nicht mit allen antigenen Peptiden oder Klasse-I-Allotypen.

Es sind Austauschverfahren bekannt, bei denen ein an ein gefaltetes Rezeptor-Protein gebundener fotosensibler Helfer-Ligand zunächst mittels UV-Licht (RODENKO B ET AL: "Generation of peptide-MHC class I complexes through UV-mediated ligand exchange",NATURE PROTOCOLS, NATURE PUBLISHING GROUP, GB, Bd. 1, Nr. 3, 1. Januar 2006, S. 1120-1131; TOEBES M ET AL: "DESIGN AND USE OF CONDITIONAL MHC CLASS I LIGANDS", NATURE MEDICINE, NATURE PUBLISHING GROUP, NEW YORK, NY,US, Bd. 12, Nr. 2, 5. Februar 2006, S. 246-251; EP 1 683 808 A1) oder auf chemischem Wege (EP 1 683 808 A1; BORIS RODENKO ET AL: "Class I major histocompatibility complexes loaded by a periodate trigger", JOURNAL OF THE AMERICAN CHEMICAL SOCIETY, ACS PUBLICATIONS, US, Bd. 131, Nr. 34, 1. August 2009, S. 13205-13213) gespalten und das dadurch entleerte Rezeptor-Protein anschließend mit einem Untersuchungspeptid neu besetzt wird.

In P. V. K. PRAVEEN ET AL ("Tapasin edits peptides on MHC class I molecules by accelerating peptide exchange", EUROPEAN JOURNAL OF IMMUNOLOGY, Bd. 40, Nr. 1, 16. Januar 2010, S. 214-224) wird der Austausch eines Peptids SIINYEKL mit vergleichsweise geringer Affinität zu einem MHC-Klasse-I-Protein gegen ein Peptid SIINFEKL-bio mit höherer Affinität in Gegenwart von Tapasin beschrieben. Tapasin ist ein natürlicherweise im Lumen des Endoplasmatischen Retikulums vorkommenden Glykoprotein aus 428 Aminosäuren, welches ein Bestandteil des an der Beladung von MHC-I-Molekülen mit antigenen Peptiden beteiligten Peptidbeladungskomplexes (PLC) ist.

In CHEN LET AL ("Endolysosomal processing of exogenous antigen into major histocompatibility complex class I-binding peptides", SCANDINAVIAN JOURNAL OF IMMUNOLOGY, Bd. 59, Nr. 6, Juni 2004, S. 545-552) werden Oktapeptide (RGY8-6h und SIINFEKL) beschrieben, derartige Peptide sind jedoch keine Ligandentauscher/Ligandenfreisetzer im Sinne der vorliegenden Erfindung.

Die Aufgabe der Erfindung ist es, die Herstellung von Klasse-I-Peptid-Komplexen zu vereinfachen.

Gelöst wird die Aufgabe durch ein Verfahren zum Herstellen eines Untersuchungsreagens, wobei einem ungefalteten Rezeptor-Protein, wobei das ungefaltete Rezeptor-Protein ein ungefaltetes MHC-Klasse-I-Protein ist, in einer Ausgangslösung ein gegen ein Untersuchungspeptid austauschbarer Helfer-Ligand zugefügt wird, der die Faltung des MHC-Klasse-I-Proteins, ermöglicht. In einer so erzeugten Vorlösung wird dem gefalteten MHC-Klasse-I-Protein, das den Helfer-Liganden gebunden aufweist, ein Ligandentauscher/Ligandenfreisetzer zugefügt, wobei der Ligandentauscher/Ligandenfreisetzer eine geringere Affinität zum MHC-Klasse-I-Protein aufweist als der Helfer-Ligand, so dass eine Analyselösung des Untersuchungsreagens vorliegt, die ein gefaltete MHC-Klasse-I-Protein mit daran gebundenem Untersuchungspeptid aufweist. Das Untersuchungspeptid wird hier ggfs. auch als "antigenes Peptid" bezeichnet.

Die Vorteile bei diesem Vorgehen liegen insbesondere darin, dass ein Untersuchungsreagens einfacher, schneller und kostengünstiger hergestellt werden kann. Dabei liegt der Erfindung der Gedanke zugrunde, einen Helfer-Liganden einzusetzen, der aus dem Komplex mit dem Rezeptor-Protein verdrängt werden kann, ohne dass eine vorherige Spaltung des Helfer-Liganden mittels z.B. energiereicher Strahlung oder aggressiver Chemikalien erforderlich wird. Erfindungsgemäß werden Konzentrations- und/oder Affinitätsunterschiede, vorzugsweise Affinitätsunterschiede ausgenutzt, um eine Verdrängung des Helfer-Liganden zu erreichen.

Das "Rezeptor-Protein" ist insbesondere ein MHC-Klasse-I-Protein.

Ein "MHC-Klasse-I-Protein" ist ein Haupthistokompatibilitätskomplex-Klasse-I-Protein (auch bezeichnet als MHC-Klasse-I-Molekül, Haupthistokompatibilitäts-Klasse-I-Protein, engl. MHC class I molecule), welches ein Transmembranprotein der zellulären Immunantwort ist. Diese MHC-Klasse-I-Proteine binden Peptide aus dem Zellinneren, wie beispielsweise aus dem Cytosol oder dem Lumen der endocytischen Organellen. Zudem präsentieren diese an der Zelloberfläche den zytotoxischen T-Zellen ein Peptid-Antigen (Antigenpräsentation). Menschliche MHC-Klasse-I-Proteine werden auch als HLA-Proteine (HLA = human leukocyte antigen) bezeichnet.

Zudem umfasst der Begriff "MHC-Klasse-I-Protein" hier auch den eigentlichen MHC-Klasse-I-Proteinen strukturell und/oder funktionell ähnliche Proteine, die ebenfalls im MHC kodiert sind und die ebenfalls Peptide binden, nämlich HLA-E, HLA-F und HLA-G. Zudem umfasst der Begriff auch Fragmente aller dieser Proteine, insbesondere die extrazelluläre Domäne, sofern diese Peptide binden. Zudem umfasst der Begriff hier sowohl die schweren Ketten ("Heavy chains", großen Untereinheiten) der MHC-Klasse-I-Proteine, als auch die in Zellen normalerweise an sie gebundene invariante kleine Untereinheit (leichte Kette, "light chain"), auch bekannt als das Protein "Beta-2 Mikroglobulin" (β2m), als auch den Komplex aus der schweren und der leichten Kette. Zudem umfasst der Begriff auch ein Fusionsprotein, das aus der schweren Kette (oder einem Fragment der schweren Kette), einem Linker (insbesondere der Sequenz Glycin-Glycin-Glycin-Serin-Glycin-Glycin-Glycin-Serin-Glycin-Glycin-Glycin-Serin-, s. SEQ ID NO: 1) und der leichten Kette besteht. Unter einem "Fragment" werden hier insbesondere zusammenhängende Teilsequenzen von mindestens 10, 15, 20, 25, 30, 35, 40, 45, 50, 60, 65, 70, 80, 90 oder mindestens 100 Aminosäuren, bevorzugt mindestens 110, 120, 130, 140, 150, 160, 170, 180, 190 oder mindestens 200 Aminosäuren verstanden.

Der Begriff "MHC-Klasse-I-Protein" umfasst auch die MHC-Klasse-I-Proteine nicht-menschlicher Wirbeltierspezies. Die MHC-Klasse-I-Proteine nicht-menschlicher Wirbeltierspezies spielen eine Rolle in der Untersuchung und Heilung von Krankheiten dieser Wirbeltierspezies, beispielsweise in der Veterinärmedizin, und in Tierexperimenten, in denen menschliche Krankheiten am Tiermodell untersucht werden, zum Beispiel der EAE (experimentellen autoimmunen Encephalomyelitis) bei Mäusen *(Mus musculus),* die ein Tiermodell für die menschliche Krankheit Multiple Sklerose darstellt. Nicht-menschliche Wirbeltierspezies sind beispielsweise und insbesondere die Maus *(Mus musculus),* die Ratte (*Rattus norvegicus*), die Kuh (*Bos taurus*), das Pferd (*Equus equus*) und die Grüne Meerkatze *(Macaca mulatta).* Die MHC-Klasse-Proteine der Maus werden beispielsweise als H-2-Proteine bezeichnet, die von den Genloci H-2K, H-2L und H-2D kodiert werden. Ein bestimmter Allotyp eines Maus-MHC-Klasse-I-Proteins ist beispielsweise H-2K^{b} oder H-2L^{d}.

Unter einem "gefalteten Protein" ist ein Protein von einer solchen Konformation (dreidimensionalen Struktur) zu verstehen, dass seine Konformation stabil (d.h. über eine Zeitspanne von Stunden hinweg nicht wesentlich veränderlich) und gleichzeitig seine biologische Aktivität (beispielsweise die Bindung eines Peptids) intakt ist. Unter einem "ungefalteten Protein" ist im Gegensatz dazu ein Protein von einer solchen Konformation zu verstehen, dass es diese beiden Kriterien nicht erfüllt. Insbesondere ist unter einem "gefalteten Protein" ein Rezeptor-Protein mit einer Konformation zu verstehen, in der das Rezeptor-Protein Peptide in vivo bindet.

Unter dem hier verwendeten Begriff "Peptid" sind hier Peptide und peptidähnliche chemische Verbindungen zu verstehen, insbesondere solche, die an MHC-Klasse-I-Proteine binden, und weiter insbesondere solche, die im Komplex mit MHC-Klasse-I-Proteinen von CTL erkannt werden.

Unter dem Begriff "Untersuchungspeptide" sind insbesondere Peptide zu verstehen, die eine hohe Affinität (d.h. eine Dissoziationskonstante K_{D} ≤ 1 µM, vorzugsweise ≤ 500 nM, bevorzugt ≤ 300nM, ≤ 200 nM oder ≤ 100nM) zum Rezeptor-Protein aufweisen. Untersuchungspeptide sind Peptide, die eine Aminosäuresequenz aufweisen, die typischerweise durch die durchzuführende Untersuchung bzw. den Anwendungsfall, beispielsweise eine zu untersuchende Krankheit oder andere diagnostische Aufgaben, bestimmt ist und von einer zur nächsten Untersuchung verschieden sein kann. Untersuchungspeptide haben typischerweise eine Länge von 8, 9, 10 oder 11, beispielsweise 9 oder 10 Aminosäuren.

Helfer-Liganden, hier gegebenenfalls synonym auch als "Helfer-Peptide" bezeichnet, sind Peptide oder peptidähnliche chemische Verbindungen, die mit hoher Affinität (d.h. mit einer Dissoziationskonstante K_{D} ≤ 1 µM) an das Rezeptor-Protein, z.B. ein MHC-Klasse-I-Protein, binden und die Faltung des MHC-Klasse-I-Proteins aus dem ungefalteten Zustand oder die Stabilisierung des gefalteten MHC-Klasse-I-Proteins bewirken oder fördern. Helfer-Liganden haben vorzugsweise eine Länge von 2, 3, 4, 5, 6, 7, 8, 9, 10, oder 11, besonders bevorzugt 8, 9, 10, oder 11, beispielsweise 8 oder 9 Aminosäuren. Helfer-Liganden weisen vorzugsweise eine geringere Affinität zum Rezeptor-Protein auf als die einzusetzenden Untersuchungspeptide. Der Unterschied beträgt vorzugsweise etwa eine Größenordnung, so dass die Dissoziationskonstante des Helfer-Liganden beispielsweise K_{D} = 500 nM beträgt, wenn die Dissoziationskonstante des Untersuchungspeptids K_{D} = 50 nM beträgt.

Der Austausch erfolgt vorzugsweise durch die Zugabe eines Ligandentauschers/Ligandenfreisetzers und des antigenen Untersuchungspeptids zu der Lösung eines RezeptorProteins oder -Proteinkomplexes, z.B. MHC-Klasse-I-Proteins oder Multimers. Der Ligandentauscher/Ligandenfreisetzer fördert den Austausch eines Helfer-Liganden durch ein Untersuchungspeptid in der Analyselösung, oder ermöglicht diesen Austausch gegebenenfalls erst, so dass das MHC-Klasse-I-Protein in der Analyselösung das Untersuchungspeptid enthält, d.h. bindet.

Vorzugsweise erfolgt der Austausch unter Inkubation bei einer Temperatur zwischen 4 °C und 40 °C für eine Zeit zwischen 1 Minute und 60 Minuten, und eine anschließende Trennung des MHC-Klasse-I-Proteins oder Multimers von dem Helfer-Liganden und ggfs. dem Ligandentauscher/Liganden-freisetzer. Diese Trennung kann insbesondere erfolgen durch Flüssigchromatographie, insbesondere Gelfiltrationschromatographie, oder Affinitätschromatograpie.

Ohne dass eine Bindung an eine Theorie beabsichtigt ist, wird angenommen, dass die Verdrängungsreaktion in Gegenwart des Ligandentauschers/Ligandenfreisetzers so abläuft, dass der Helfer-Ligand im Verlauf der Gleichgewichtsreaktion zwischen Bindung und Bindungslösung einen Teil der Peptidbindungsstelle des MHC-Klasse-I-Proteins freigibt oder von ihr dissoziiert, und dass im nächsten Schritt der Ligandentauscher/Ligandenfreisetzer diesen Teil der Peptidbindungsstelle des MHC-Klasse-I-Proteins besetzt und/oder an ihn bindet, und dass im nächsten Schritt der Helfer-Ligand nicht wieder an den entsprechenden Teil der Peptidbindungsstelle des MHC-Klasse-I-Proteins binden kann und daher nur teilweise oder mit einem Teil seiner Struktur an das MHC-Klasse-I-Protein gebunden bleibt, dass der Helfer-Ligand sich anschließend vollständig von der Peptidbindungsstelle des MHC-Klasse-I-Proteins ablöst und dadurch eine freie Peptidbindungsstelle des MHC-Klasse-I-Proteins entsteht, an die im nächsten Schritt das Untersuchungspeptid bindet, wodurch ein Komplex aus einem MHC-Klasse-I-Protein und dem Untersuchungspeptid entsteht.

Erfindungsgemäß ist oder umfasst der "Ligandentauscher/Ligandenfreisetzer" eine Aminosäure oder eine modifizierte Aminosäure.

Eine Aminosäure ist eine chemische Verbindung, die eine mit einem Kohlenstoff (C)-Atom verbundene Amino- oder Ammoniumgruppe (-NH₂ oder -NH₃⁺) und gleichzeitig eine mit demselben oder einem anderen Kohlenstoffatom verbundene Carboxyl- oder Carboxylatgruppe (-COOH oder -COO⁻) enthält. Beispiele für eine Aminosäure sind Glycin, Alanin, Homoleucin, Beta-Alanin, und ε-Acetyl-Lysin. Von einer modifizierten Aminosäure wird vorliegend dann ausgegangen, wenn die Aminogruppe mit einer anderen chemischen Verbindung (zum Beispiel einer Carbonsäure oder einem ihrer Derivate) derart umgesetzt ist, dass diese andere chemische Verbindung kovalent an die Aminogruppe gebunden ist, zum Beispiel durch eine Amidbindung (Peptidbindung, Lactambindung) oder eine einer Amidbindung ähnliche Bindung (zum Beispiel eine Thioamidbindung). Von einer modifizierten Aminosäure wird vorliegend auch dann ausgegangen, wenn die Carboxylgruppe mit einer anderen chemischen Verbindung (zum Beispiel einem Alkohol, einem Amin, oder einem ihrer Derivate) derart umgesetzt ist, dass diese andere chemische Verbindung kovalent an die Carboxylgruppe gebunden ist, zum Beispiel durch eine Esterbindung oder durch eine Amidbindung oder durch eine einer Ester- oder Amidbindung ähnliche Bindung. Von einer modifizierten Aminosäure wird vorliegend auch dann ausgegangen, wenn sowohl die Aminogruppe als auch die Carboxylgruppe in dem in diesem Abschnitt beschriebenen Sinne umgesetzt worden sind. Beispiele von modifizierten Aminosäuren sind Acetylmethionin, Alaninamid und N-acetyltryptophanamid.

Erfindungsgemäß ist der Ligandentauscher/Ligandenfreisetzer ein Peptid aus 2 oder 3 Aminosäuren.

Auch kann der Ligandentauscher/Ligandenfreisetzter ein Peptidsein oder ein Peptid umfassen, welches dadurch charakterisiert ist, dass es aus mehreren Aminosäuren besteht, die wie in einem Protein durch Amidbindungen (Peptidbindungen) miteinander verbunden sind, oder die durch Amidbindungen ähnliche Bindungen, z.B. Thioamidbindungen, miteinander verbunden sind. Von einem Peptid wird vorliegend auch dann ausgegangen, wenn die aminoterminale Aminogruppe oder die carboxyterminale Carboxylgruppe oder das Stickstoffatom einer zuvor beschriebenen internen Amidbindung mit anderen Verbindungen umgesetzt worden sind.

Weiterhin kann "der Ligandentauscher/Ligandenfreisetzer eine peptidähnliche chemische Verbindung umfassen oder sein. "Peptidähnliche chemische Verbindungen" sind beispielsweise chemische Verbindungen, in denen die internen Peptidbindungen (Amidbindungen) durch Lactame oder Piperazinone ersetzt sind, und auch 7,6-bizyklisches Pyridizin, 9-aminooctahydro-6,10-dioxo-6H-pyridazino[1,2-a][1,2]diazepin-1-carbonsäure, 5-amino-1,2,4,5,6,7-hexahydroazepino[3,2,1-*hi*]indol-4-on-2-carbonsäure, und methylbutyl-substituierte Lactame, insbesondere 1-(*tert-*butyloxycarbonyl)-7-[1-(tert-butyloxycarbonyl)-3-methylbutyl]-6-oxo-1,7-diazaspiro[4.5]dekan.

Bevorzugte Ligandentauscher/Ligandenfreisetzer sind Peptide, die aus 1, 2, 3, 4, 5, 6, oder 7 Aminosäuren, besonders bevorzugt 2 oder 3 Aminosäuren, bestehen, wovon vorzugsweise eine Aminosäure, insbesondere die am Carboxyl-Terminus des Peptids, eine modifizierte Aminosäure ist.

Der Ligandentauscher/Ligandenfreisetzer bindet vorzugsweise mit niedrigerer Affinität an das Rezeptor-Protein, z.B. das MHC-Klasse-I-Protein, als das Untersuchungspeptid und besonders bevorzugt auch mit niedriger Affinität als der Helfer-Ligand. Beispielsweise kann die Dissoziationskonstante K_{D} eines Untersuchungspeptids 100 nM betragen, während die Dissoziationskonstante K_{D} des Ligandentauschers/Liganden-freisetzers 10 µM bis 10 mM, beispielsweise 1 mM, beträgt. Der Unterschied in der Bindungsaffinität zwischen dem Untersuchungspeptid und dem Ligandentauscher/Ligandenfreisetzer beträgt vorzugsweise mindestens etwa zwei Größenordnungen (Faktor 100). Der Helfer-Ligand kann eine dazwischen liegende Bindungsaffinität, beispielsweise eine Dissoziationskonstante von 1 µM aufweisen. Eine niedrigere Bindungsaffinität des Ligandentauschers/Ligandenfreisetzers kann beispielsweise dadurch erzielt werden, dass ein Peptid verwendet wird, das kürzer ist als das Untersuchungspeptid. Beispielsweise kann der Ligandentauscher/Ligandenfreisetzer eine einzelne Aminosäure sein oder ein Peptid, das aus 2, 3, 4, 5, 6, oder 7 Aminosäuren, bevorzugt aus 2 oder 3 Aminosäuren besteht. Wenn ein Helfer-Ligand und ein Ligandentauscher/Ligandenfreisetzer zusammen eingesetzt werden, ist eine Kombination besonders bevorzugt, bei der der Helfer-Ligand ein Peptid aus 8, 9, 10 oder 11 Aminosäuren, beispielsweise 9 oder 10 Aminosäuren, ist und der Ligandentauscher/Ligandenfreisetzer eine einzelne Aminosäure oder ein Peptid aus 2, 3, 4, 5, 6, oder 7, bevorzugt 2 oder 3 Aminosäuren ist. Der Begriff "Peptid" umfasst auch in diesem Zusammenhang peptidähnliche Verbindungen.

Das einen Helfer-Liganden aufweisende Rezeptor-Protein hat insbesondere folgenden Aufbau: Ein MHC-Klasse-I-Protein im gefalteten Zustand, wobei der Helfer-Ligand in/an der Peptidbindungsstelle des MHC-Klasse-I-Proteins gebunden ist.

Um eine effektive und große Ausbeute an ungefalteten MHC-Klasse-I-Proteinen zu gewährleisten, kann das ungefaltete MHC-Klasse-I-Protein in einem Expressionssystem hergestellt werden.

"Expression" ist die Herstellung eines Proteins, insbesondere eines MHC-Klasse-I-Proteins (wie zuvor beschrieben, d.h. sowohl der großen als auch der kleinen Untereinheit, gemeinsam, getrennt, oder miteinander verbunden), in einem Expressionssystem.

In einer diesbezüglichen Ausführungsform kann das Expressionssystem eine Bakterienzelle aufweisen, insbesondere eine *Escherichia-coli-*Zelle, oder eine Hefezelle, insbesondere eine *Saccharomyces-cerevisiae-*Zelle, oder eine Insektenzelle, insbesondere eine *Spodoptera-frugiperda-*Zelle, oder eine Säugerzelle, insbesondere eine CHO-Zelle, wobei CHO für "Chinese Hamster (*Cricetulus griseus)* Ovary" steht, oder ein zellfreies Expressionssystem, insbesondere als Retikulozyten-Lysat.

Um eine möglichst hohe Ausbeute zu erhalten, weist das Expressionssystem mehrere Zellen auf, insbesondere eine Anzahl von Zellen in einer Größenordnung von mindestens 1.000.000 Zellen.

Unter dem "Untersuchungsreagens" ist insbesondere eine Verbindung, Substanz, Komplex oder ein Partikel zu verstehen, die/das an einen TCR einer CTL spezifisch bindet und die/das für eines der Zwecke, die in der Einleitung beschrieben sind, verwendet wird, insbesondere für den Nachweis einer T-Zellen-Frequenz, für die positive Selektion (Isolierung) von CTL und für die negative Selektion (Entfernung) von CTL. Untersuchungsreagenzien sind insbesondere Multimere wie sie im Weiteren beschrieben werden, Partikel (gemäß der späteren Erläuterung zum Multimerisierungselement) und Membranpräparationen wie sie später beschrieben werden. Der Begriff "Untersuchungsreagens" umfasst insbesondere ein gefaltetes Rezeptor-Protein, das ein Untersuchungspeptid gebunden aufweist, ein Multimer umfassend mindestens zwei solche gefaltete Rezeptor-Proteine mit gebundenem Untersuchungspeptid und ein Substrat, z.B. ein Partikel, in, an oder auf dem mindestens ein solches Rezeptor-Protein oder Multimer immobilisiert ist.

"Spezifische Bindung" ist dabei eine Bindung, die deutlich stärker ist als die durchschnittlich beobachtbare ("nichtspezifische") Bindung zwischen einem zufällig ausgewählten TCR und einem zufällig ausgewählten MHC-Klasse-I-Protein, wobei "deutlich" bedeutet, dass die Dissoziationskonstante K_{D} der spezifischen Bindung mindestens zehnmal niedriger als die einer nichtspezifischen Bindung ist.

In einer weiteren Ausführungsform wird dem MHC-Klasse-I-Protein ein Signalgeber zugeordnet, wobei der Signalgeber insbesondere ein Fluoreszenzfarbstoff ist. Somit kann eine einfache Methode zur Identifizierung des MHC-Klasse-I-Proteins bereitgestellt werden. "Zugeordnet" heißt hier kovalent oder nicht-kovalent gebunden.

Weiterhin kann dem MHC-Klasse-I-Protein ein Fusionselement zugeordnet werden. Unter einem Fusionselement ist eine Polypeptidsequenz zu verstehen, die eine Proteindomäne oder einen Teil davon umfasst. Beispiele für Fusionselemente sind insbesondere die F_{c}-Domänen von Antikörpern, *Staphylococcus aureus* Protein A, oder eine Oligohistidin- oder Oligoarginin-Sequenz, wobei eine Oligohistidin-Sequenz aus drei bis zwölf, vorzugsweise sechs, Histidinen besteht, oder solche Proteindomänen, die stabile *Coiled-coil*-Wechselwirkungen miteinander eingehen, insbesondere die helikalen Domänen der Transkriptionsfaktoren *jun* und *fos.* "Zugeordnet" heißt hier kovalent oder nicht-kovalent gebunden.

Vorteil des Fusionselements ist, dass es die spezifische Bindung des MHC-Klasse-I-Proteins an ein Multimerisierungselement oder an ein Partikel erlaubt. Der Vorteil des Fusionselements liegt außerdem darin, dass es die Reinigung und den Nachweis von MHC-Klasse-I-Proteinen mit Hilfe solcher Techniken ermöglicht, bei denen das Fusionselement durch spezifisch daran bindende Reagenzien spezifisch erkannt wird. Ein weiterer Vorteil des Fusionselements liegt darin, dass es die direkte Bindung von MHC-Klasse-1-Proteinen aneinander ermöglicht.

Weiterhin kann dem Rezeptor-Protein ein Ankerelement zugeordnet werden. Dieses Ankerelement ist insbesondere ein Biotinmolekül, welches entweder durch eine natürliche Biotinylierungssequenz, die in dem Gen für das MHC-Klasse-I-Protein genetisch kodiert ist, und ein biotinylierendes Enzym, insbesondere BirA, oder durch chemische Methoden, insbesondere Verwendung eines N-hydroxy-succinimid-Derivats von Biotin, an dem Protein angebracht wird. "Zugeordnet" heißt hier vorzugsweise gebunden, beispielsweise kovalent oder nicht-kovalent gebunden.

Der Vorteil des Ankerelements liegt insbesondere darin, dass es die Zusammenfügung von MHC-Klasse-I-Proteinen zu Multimeren und die Bindung an ein Partikel ermöglicht. Der Vorteil des Ankerelements liegt außerdem darin, dass es die Reinigung und den Nachweis von MHC-Klasse-I-Proteinen mit Hilfe solcher Techniken ermöglicht, bei denen das Ankerelement durch spezifisch daran bindende Reagenzien spezifisch erkannt wird.

Um den Aviditätseffekt auszunutzen, können mehrere gefaltete MHC-Klasse-I-Proteine zu einem Multimer zusammengefügt werden.

Das Zusammenfügen der MHC-Klasse-I-Proteine zu einem Multimer kann beispielsweise dadurch erfolgen, dass die MHC-Klasse-I-Proteine mit einem Multimerisierungselement zusammengebracht werden.

Das "Multimerisierungselement" ist dadurch gekennzeichnet, dass die Ankerelemente, die an den MHC-Klasse-I-Proteinen vorhanden sind, mit dem Multimerisierungselement eine nicht-kovalente oder kovalente Bindung eingehen.

Ein solches "Multimerisierungselement" ist insbesondere Avidin oder Streptavidin.

In anderen Fällen kann ein solches Multimerisierungselement ein Partikel sein, beispielsweise vom Durchmesser von 1 Mikrometer bis 1 Millimeter, beispielsweise aus Agarose bestehend, welches solcherart chemisch modifiziert ist, dass die Oberfläche des Partikels an die Ankerelemente spezifisch binden kann. Eine solche chemische Modifikation der Oberfläche kann durch die kovalente oder nichtkovalente Bindung von z.B. Avidin oder Streptavidin oder durch die Bindung eines Antikörpers, der mit einem Teil des MHC-Klasse-I-Proteins reagiert, an die Oberfläche erfolgen.

Die Zusammenfügung mehrerer MHC-Klasse-I-Proteine zu einem Multimer kann auch dadurch erfolgen, dass die MHC-Klasse-I-Proteine miteinander eine Bindung eingehen, ohne dass ein Bindeprotein erforderlich ist.

Das "Multimer" umfasst insbesondere ein Tetramer und/oder ein Pentamer und weist insbesondere zwei oder mehr MHC-Klasse-I-Proteine auf.

Der Vorteil des Multimers liegt unter anderem darin, dass das Multimer durch Filtration oder Chromatographie erhaltbar sein kann und dass mindestens zwei MHC-Klasse-I-Proteine an das Multimer angekoppelt sind. Ein weiterer Vorteil des Multimers liegt in dem sogenannten Aviditätseffekt, der darin besteht, dass mehrere MHC-Klasse-I-Proteine fester, d.h., mit geringerer Dissoziationskonstante K_{D}, an die TCR auf der Oberfläche einer CTL binden. Durch diese festere Bindung wird insbesondere die Detektion und Isolation von CTL erleichtert. Zudem kann durch die festere Bindung eine Aktivierung der CTL herbeigeführt werden.

Unter "Aktivierung" der CTL ist dabei zu verstehen, dass durch die Bindung von Proteinen an die TCR der CTL, insbesondere durch die Bindung von Untersuchungsreagenzien, insbesondere MHC-Klasse-I-Proteinen, Multimeren, Partikeln oder Membranpräparationen, Stoffwechselvorgänge im Inneren der CTL verändert werden.

Um eine einfache Methode zur Identifizierung bereitzustellen, kann dem Multimer ein Multimersignalgeber zugeordnet werden, wobei der Multimersignalgeber insbesondere ein Fluoreszenzfarbstoff ist. "Zugeordnet" bedeutet insbesondere die Bindung, beispielsweise die kovalente oder nicht-kovalente Bindung des Multimersignalgebers an das Multimer.

Weiterhin können die MHC-Klasse-I-Proteine in Membranpräparationen multimerisiert werden. Unter "Membranpräparationen" sind dabei Lipidmembranen zu verstehen, die dadurch gekennzeichnet sind, dass sie MHC-Klasse-I-Proteine enthalten. Die MHC-Klasse-I-Proteine können dadurch enthalten sein, dass sie teilweise in die Lipidmembran eintauchen und durch hydrophobe Wechselwirkungen in oder an der Lipidmembran festgehalten werden; oder sie können dadurch enthalten sein, dass sie an die Oberfläche der Lipidmembranen kovalent oder nichtkovalent gebunden sind.

Unter "Lipidmembranen" sind solche Membranen zu verstehen, die aus der Zelloberfläche (Plasmamembran) von eukaryotischen Zellen bestehen oder in Bau und Zusammensetzung der Plasmamembran im Wesentlichen entsprechen; dabei ist es unerheblich, ob die Zellen lebendig oder fixiert (tot), intakt (durchlässig für wasserlösliche Substanzen) oder nicht intakt sind. Darunter sind auch solche Membranen zu verstehen, die aus isolierten Bestandteilen, insbesondere Lipiden, im Labor zusammengefügt worden sind.

Die Einsetzung der MHC-Klasse-I-Proteine in die Lipidmembranen kann dadurch erfolgen, dass die MHC-Klasse-I-Proteine in einer Zelle (z.B. in einem Expressionssystem) hergestellt werden und dass die Membranpräparation aus dieser Zelle hergestellt wird. Die Einsetzung der MHC-Klasse-I-Proteine in die Lipidmembranen kann auch dadurch erfolgen, dass die MHC-Klasse-I-Proteine zu einer vorher existierenden Lipidmembran hinzugefügt werden.

Der Vorteil der Membranpräparation liegt unter anderem darin, dass die Membranpräparation aus Expressionssystemen erhaltbar sein kann und dass mindestens zwei MHC-Klasse-I-Proteine in der Membranpräparation enthalten sind. Ein weiterer Vorteil der Membranpräparation liegt in dem so genannten Aviditätseffekt, der darin besteht, dass mehrere MHC-Klasse-I-Proteine fester, d.h., mit geringerer Dissoziationskonstante K_{D}, an die TCR auf der Oberfläche einer CTL binden. Durch diese festere Bindung wird insbesondere die Detektion und Isolation von CTL erleichtert. Zudem kann durch die festere Bindung eine Aktivierung der CTL herbeigeführt werden.

Weiterhin kann die Aufgabe auch durch ein gefaltetes Rezeptor-Protein gelöst werden, insbesondere ein gefaltetes MHC-Klasse-I-Protein, welches einen Helfer-Liganden aufweist. Durch Zugabe des Untersuchungspeptids und vorzugsweise zusätzlich des Ligandentauschers/Ligandenfreisetzers kann in vorteilhafter Weise ein Rezeptor-Protein, z.B. ein MHC-Klasse-I-Protein, bereitgestellt werden, welches das zugegebene Untersuchungspeptid im Austausch gegen den Helfer-Liganden bindet und im Anschluss daran das Untersuchungspeptid aufweist.

Der Helfer-Ligand ist bevorzugt ein Peptid aus 2, 3, 4, 5, 6, 7, 8, 9, 10, oder 11, weiter bevorzugt 8, 9, 10 oder 11, besonders bevorzugt 9 oder 10 Aminosäuren.

Vorzugsweise weist das gefaltete Protein neben dem Helfer-Liganden oder anstelle des Helfer-Liganden einen Ligandentauscher/Ligandenfreisetzer auf, der eine geringere Affinität zum Rezeptor-Protein als der Helfer-Ligand aufweist und/oder eine Aminosäure oder ein Peptid aus 2, 3, 4, 5, 6, oder 7, bevorzugt 2 oder 3 Aminosäuren ist. Besonders bevorzugt ist eine Kombination, bei der der Helfer-Ligand ein Peptid aus 8, 9, 10 oder 11, beispielsweise 9 oder 10 Aminosäuren ist und der Ligandentauscher/Ligandenfreisetzer eine einzelne Aminosäure oder ein Peptid aus 2, 3, 4, 5, 6, oder 7, bevorzugt 2 oder 3 Aminosäuren ist.

Ebenso kann die die Aufgabe gelöst werden durch ein Multimer, welches gefaltete MHC-Klasse-I-Proteine aufweist, welche einen Helfer-Liganden und vorzugsweise auch oder alternativ einen erfindungsgemäßen Ligandentauscher/Ligandenfreisetzer aufweisen. Durch Zugabe des Untersuchungspeptids und vorzugsweise des Ligandentauschers/Ligandenfreisetzers kann vorteilhafter Weise ein Multimer bereitgestellt werden, welches das zugegebene Untersuchungspeptid im Austausch gegen den Helfer-Liganden bindet und im Anschluss daran zumindest teilweise das Untersuchungspeptid aufweist. Durch die Verwendung eines Multimers kann vorteilhafter Weise der Aviditätseffekt ausgenutzt werden, der es ermöglicht, dass das Multimer an die Oberfläche der CTL bindet.

Auch kann die Aufgabe gelöst werden durch ein Partikel, welches gefaltete MHC-Klasse-I-Proteine aufweist, welche Helfer-Liganden aufweisen. Durch Zugabe des Ligandentauschers/Ligandenfreisetzers und des Untersuchungspeptids kann vorteilhafter Weise ein Partikel bereitgestellt werden, welcher das zugegebene Untersuchungspeptid im Austausch gegen den Helfer-Liganden bindet und im Anschluss daran zumindest teilweise das Untersuchungspeptid aufweist. Durch die Verwendung eines Partikels kann vorteilhafter Weise der Aviditätseffekt ausgenutzt werden, der es ermöglicht, dass das Multimer an die Oberfläche der CTL bindet.

Weiterhin kann die Aufgabe gelöst werden durch eine Membranpräparation, welche gefaltete MHC-Klasse-I-Proteine aufweist, welche einen Helfer-Liganden aufweisen. Durch Zugabe des Ligandentauschers/Ligandenfreisetzers und des Untersuchungspeptids kann vorteilhafter Weise eine Membranpräparation bereitgestellt werden, welche das zugegebene Untersuchungspeptid im Austausch gegen das Helfer-Peptid bindet und im Anschluss daran zumindest teilweise das Untersuchungspeptid aufweist. Durch die Verwendung einer Membranpräparation kann vorteilhafter Weise der Aviditätseffekt ausgenutzt werden, der es ermöglicht, dass das Multimer an die Oberfläche der CTL bindet.

Die hier verwendeten Begrifflichkeiten entsprechen dem zuvor erarbeiteten Verständnis.

Um einerseits eine effiziente Faltung des MHC-Klasse-I-Proteins und andererseits einen effizienten Austausch des Helfer-Liganden gegen das Untersuchungspeptid zu erreichen, kann der Helfer-Ligand eine modifizierte Aminosäure oder ein Peptid sein, welches einen Bestandteil eines austauschfähigen und gefalteten MHC-Klasse-I-Proteinkomplexes darstellt. Insbesondere wird ein gefaltetes MHC-Klasse-I-Protein gebildet, das den Helfer-Liganden aufweist.

Der "austauschfähige MHC-Klasse-I-Proteinkomplex" umfasst ein gefaltetes MHC-Klasse-I-Protein, das das Helfer-Peptid aufweist. "Austauschfähig" bedeutet die Eigenschaft eines Rezeptorproteins oder Rezeptorproteinkomplexes, dass ein an einer Bindungsstelle des Rezeptorproteins oder Rezeptorproteinkomplexes gebundener Helfer-Ligand gegen ein Untersuchungspeptid ausgetauscht werden kann.

Ein "Vorlösungsuntersuchungsreagens" umfasst einen austauschfähigen Rezeptorproteinkomplex, z.B. MHC-Klasse-I-Proteinkomplex, d.h. ein gefaltetes Rezeptor-Protein, das einen Helfer-Liganden gebunden aufweist, oder in einer anderen Ausprägung ein Multimer, das gefaltete Rezeptorproteine, z.B. MHC-Klasse-I-Proteine, aufweist, die den Helfer-Liganden aufweisen, oder in einer anderen Ausprägung ein Partikel, das gefaltete Rezeptorproteine, z.B. MHC-Klasse-I-Proteine aufweist, die den Helfer-Liganden aufweisen, oder in einer anderen Ausprägung eine Membranpräparation, die gefaltete Rezeptorproteine, z.B. MHC-Klasse-I-Proteine aufweist, die den Helfer-Liganden aufweisen. Ein Vorlösungsuntersuchungsreagens umfasst des weiteren vorzugsweise einen Ligandentauscher/Ligandenfreisetzer.

Die "gefalteten MHC-Klasse-I-Proteine" sind durch die bereits erfolgte Definition bestimmt.

Ebenfalls kann die Aufgabe gelöst werden durch eine "Vorlösung", welche ein Vorlösungsuntersuchungsreagenz aufweist. Somit kann eine effektive Untersuchungsmethode bereitgestellt werden. Somit kann außerdem ein Kit mit einem Vorlösungsuntersuchungsreagenz nach Vorschriften, die nach dem jetzigen Stand der Technik bereits bekannt sind, hergestellt werden.

In einem weiteren Aspekt der Erfindung kann die Aufgabe gelöst werden durch ein Untersuchungsreagens, welches wie zuvor beschrieben hergestellt ist. Somit kann eine effektive Untersuchungsmethode bereitgestellt werden. Somit kann ebenfalls eine effektive Methode bereitgestellt werden, T-Zellen zu isolieren. Somit kann ebenfalls eine effektive Methode bereitgestellt werden, T-Zellen aus einer Population zu entfernen. Somit ist außerdem für die Herstellung eines Untersuchungsreagens aus dem Vorlösungsuntersuchungsreagenz nur ein einfacher, schneller und billiger Arbeitsschritt erforderlich, der auch von einer weniger qualifizierten Person, beispielsweise einem/r Laboranten/in oder einem/r technischen Angestellten leicht durchgeführt werden kann.

Ebenfalls kann die Aufgabe gelöst werden durch eine Analyselösung, welche ein Untersuchungsreagens aufweist. Somit kann eine effektive Untersuchungsmethode bereitgestellt werden. Somit kann ebenfalls ein Kit bereitgestellt werden, der die Analyselösung enthält.

Weiterhin kann die Aufgabe gelöst werden durch einen Kit, insbesondere zum Analysieren einer T-Zellen-Frequenz, welcher ein Untersuchungspeptid und separat ein Vorlösungsuntersuchungsreagenz enthält, welches einen austauschfähigen MHC-Klasse-I-Proteinkomplex mit gebundenem Helfer-Liganden und vorzugsweise einen Ligandentauscher/Ligandenfreisetzer aufweist. Somit kann durch Austausch des Helfer-Liganden für ein Untersuchungspeptid vorzugsweise mittels des Ligandentauschers/Ligandenfreisetzers schnell, einfach und günstig ein Untersuchungsreagens hergestellt werden. Somit kann für den Laboralltag einem Laboranten ein Werkzeug zur Verfügung gestellt werden, mit dem eine einfache, schnelle und kostengünstige Herstellung eines Untersuchungsreagenz, d.h. eines MHC-Klasse-I-Proteins, Multimers, Partikels, oder einer Membranpräparation mit gebundenem Untersuchungspeptid, realisierbar ist.

Der "Kit" ist insbesondere ein System, welches einen Satz Teile aufweist, die die Durchführung einer vollständigen Analyse, wie beispielsweise die Analyse einer T-Zellen-Frequenz, vorzugsweise ohne weitere ggfs. zu erwerbende Hilfsmittel ermöglicht.

Unter "T-Zellen-Frequenz" ist derjenige Prozentsatz der CTL zu verstehen, der ein bestimmtes Peptid im Komplex mit einem bestimmten MHC-Klasse-I-Protein erkennt. Unter "CTL" sind dabei die in der Einleitung definierten Zellen zu verstehen. Die T-Zellen-Frequenz für ein Peptid, gegen das eine Immunantwort stattfindet, liegt dabei typischerweise im Bereich von 0,01% und 20%.

Auch kann die Aufgabe gelöst werden durch ein Verfahren zum Erhalt eines Analyseergebnisses, wobei ein Untersuchungspeptid, ein austauschfähiges Rezeptor-Protein oder ein austauschfähiger Rezeptorproteinkomplex, z.B. ein austauschfähiger Klasse-I-Proteinkomplex, der einen Helfer-Liganden enthält, und vorzugsweise zusätzlich der Ligandentauscher/Ligandenfreisetzer unter Bedingungen zusammengegeben werden, unter denen der Helfer-Ligand durch das Untersuchungspeptid ersetzt wird.

Bei dem Verfahren werden das Untersuchungspeptid und das austauschfähige Rezeptor-Protein vorzugsweise zusammen mit einem Ligandentauscher/Ligandenfreisetzer zusammengegeben, wobei der Helfer-Ligand bevorzugt eine geringere Affinität zum Rezeptor-Protein aufweist als das Untersuchungspeptid und der Ligandentauscher/Ligandenfreisetzer eine geringere Affinität zum Rezeptor-Protein aufweist als der Helfer-Ligand, und/oder wobei der Helfer-Ligand ein Peptid aus 2, 3, 4, 5, 6, 7, 8, 9, 10, oder 11, bevorzugt 8, 9, 10 oder 11, besonders bevorzugt 9 oder 10 Aminosäuren ist, und/oder wobei der Ligandentauscher/Ligandenfreisetzer eine Aminosäure oder ein Peptid aus 2, 3, 4, 5, 6, oder 7, bevorzugt 2 oder 3 Aminosäuren ist. Besonders bevorzugt ist eine Kombination, bei der der Helfer-Ligand ein Peptid aus 8, 9, 10 oder 11 Aminosäuren, bevorzugt 9 oder 10 Aminosäuren, ist und der Ligandentauscher/Ligandenfreisetzer eine einzelne Aminosäure oder ein Peptid aus 2, 3, 4, 5, 6, oder 7, bevorzugt 2 oder 3 Aminosäuren ist.

Durch das Analyseergebnis kann beispielsweise ein Arzt eine Diagnose bestätigen oder ausschließen.

In einer weiteren Ausprägung des Verfahrens, kann die Analyselösung mit einer Zellprobe zusammengeführt werden, sodass die Analyselösung mit einem spezifischen Element der Zellprobe komplexiert, sodass ein Komplex vorliegt. Unter einem "spezifischen Element der Zellprobe" wird ein Element verstanden, dass mit dem Rezeptorprotein oder Rezeptorproteinkomplex komplexiert. Beispielsweise kann es sich um bestimmte T-Zellen handeln.

Somit können Zellen spezifisch untersucht werden.

Dabei umfasst der Begriff "Zellprobe" insbesondere Blutproben von Menschen, Tieren, insbesondere Säuger, bis zum Knorpelfisch, und insbesondere Proben von Lymphozyten, die aus Blutproben isoliert worden sind.

"Komplexieren" ist dabei insbesondere das spezifische Binden des Reagens durch Wechselwirkungen zwischen den atomaren Elementen von Reagens und Zellprobe, insbesondere ionische Bindungen, hydrophobe Wechselwirkungen und Wasserstoffbrücken. Im einfachsten Falle bedeutet dies die spezifische Bindung eines MHC-Klasse-I-Proteins mit einem Peptid an einen TCR einer CTL. In einem anderen Fall bedeutet dies die gleichzeitige Bindung mehrerer MHC-Klasse-I-Proteine - die auch in einem Multimer enthalten sein können - an mehrere TCR einer CTL, wodurch als besonderer Vorteil der sogenannte "Aviditätseffekt" ausgenutzt wird, der darin besteht, dass die Bindung von mehreren Klasse I-Proteinen an mehrere TCRs zu einer stärkeren Bindung des Multimers an die CTL führt als die Bindung eines einzelnen MHC-Klasse-I-Proteins an einen einzelnen TCR.

Beim "Zusammenführen" werden die Analyselösung und die Zellprobe dergestalt zusammengebracht, dass eine Komplexierungsreaktion erfolgen kann. Im einfachsten Fall wird die Zellprobe in die Analyselösung gegeben.

In einer diesbezüglichen Ausführungsform erfolgt nach dem Zusammenführen ein Detektieren des Signalgebers, insbesondere durch eine Durchflusszytometrie. Dabei werden nur diejenigen Signalgeber detektiert, die mit den Zellen komplexiert sind. Somit werden diejenigen Zellen detektiert, die mit dem Signalgeber komplexiert sind.

"Detektieren" umfasst dabei sämtliche messbaren physikalischen Parameter. Insbesondere optische/ spektroskopische Verfahren sind umfasst.

Somit ergeben sich folgende Vorteile: Alle diejenigen Zellen, die mit dem Signalgeber komplexiert sind, werden detektiert. Auf diese Weise kann angegeben werden, welcher Prozentsatz der Zellen mit dem Signalgeber komplexiert ist. Auf diese Weise kann angegeben werden, welcher Prozentsatz der CTL mit einem bestimmten MHC-Klasse-I-Protein, das ein bestimmtes Untersuchungspeptid enthält, reagiert. Auf diese Weise kann die T-Zellen-Frequenz angegeben werden.

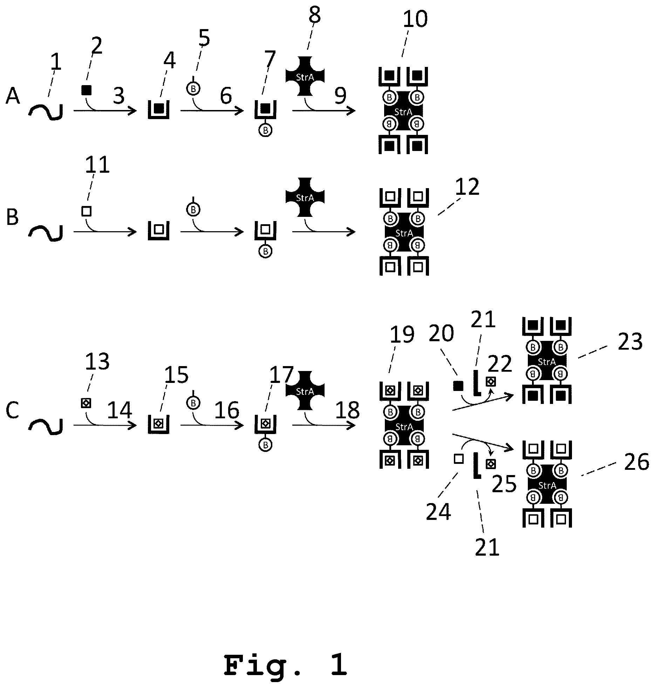

Im Weiteren wird die Erfindung anhand von Ausführungsbeispielen und der angefügten Figuren rein zu Veranschaulichungszwecken näher erläutert. Es zeigen
- Figur 1A: eine schematische Darstellung des Prinzips der Herstellung eines Multimers (hier: Tetramers) von MHC-Klasse-I-Proteinen im Komplex mit einem Untersuchungspeptid (2) nach dem Stand der Technik,
- Figur 1B: eine schematische Darstellung des Prinzips der Herstellung eines Multimers (hier: Tetramers) von MHC-Klasse-I-Proteinen nach dem Stand der Technik wie in Figur 1A, aber mit einem anderen Untersuchungspeptid (11) und
- Figur 1C: eine schematische Darstellung des Prinzips der Herstellung zweier Multimere (hier: Tetramere) von MHC-Klasse-I-Proteinen, mit verschiedenen Untersuchungspeptiden (20 und 24), mit Hilfe der erfindungsgemäßen Methode.

Die Figuren 1 und 2 zeigen schematisch die Herstellung eines Komplexes aus einem MHC-Klasse-I-Protein-Tetramer und einem Untersuchungspeptid nach dem Stand der Technik. Das entfaltete MHC-Klasse-I-Protein 1 wird mit dem Untersuchungspeptid 2, 11 versehen, und in einem Faltungsprozess 3 entsteht das gefaltete MHC-Klasse-I-Protein 4 mit gebundenem Peptid. Hiernach findet vorzugsweise ein Reinigungsschritt, insbesondere eine Gelfiltrationschromatographie, statt. Ein Ankerelement 5 oder eine Vorstufe des Ankerelements 5, z.B. Biotin (B), wird zugegeben und an das Klasse-I-Protein gebunden (6), so dass ein Klasse-I-Protein 7 mit gebundenem Peptid und Ankerelement erhalten wird. Hiernach findet vorzugsweise erneut ein Reinigungsschritt, insbesondere eine Gelfiltrationschromatographie, statt. Im nächsten Schritt wird ein Multimerisierungselement 8, z.B. Streptavidin (StrA), zugegeben, so dass ein Multimer 10, 12 erhalten wird, das das jeweilige Untersuchungspeptid 2, 11 gebunden aufweist. Hiernach findet vorzugsweise erneut ein Reinigungsschritt, insbesondere eine Gelfiltrationschromatographie, statt.

Der Stand der Technik ist dadurch gekennzeichnet, dass, um Multimere mit verschiedenen Peptiden (z.B. 10, 12) zu erhalten, man die im vorherigen Abschnitt [97] beschriebene Prozedur für jedes Multimer getrennt, von Anfang bis Ende, durchführen muss.

In Figur 1C ist ein Verfahren gemäß der Erfindung schematisch beschrieben.

Ein Helfer-Ligand 13 wird in die Faltungsreaktion 14 eingebracht und ermöglicht die Faltung eines MHC-Klasse-I-Proteins aus dem denaturierten Zustand ohne Gegenwart eines Untersuchungspeptids, so dass ein gefaltetes MHC-Klasse-I-Protein 15 mit gebundenem Helfer-Liganden resultiert. Die Schritte 16, 18 der Anbringung des Ankerelements (16) und der Herstellung des Multimers (18), inklusive der dabei ggfs. erforderlichen Reinigungen, finden anschließend wie oben für den Stand der Technik beschrieben statt. Es wird dadurch zunächst ein mit einem Ankerelement (z.B. Biotin) und dem Helfer-Liganden 13 versehenes gefaltetes MHC-Klasse-I-Protein 17, und im Anschluss an den mit Hilfe des Multimerisierungselements durchgeführten Multimerisierungsschritt 18 ein Multimer 19 erhalten, das MHC-Klasse-I-Proteine aufweist, die den Helfer-Liganden 13 aufweisen. Dieses Multimer 19 kann dann weiterverarbeitet oder als Bestandteil beispielsweise eines Kits vertrieben werden.

Um ein Multimer mit einem gewünschten Untersuchungspeptid zu erhalten, wird das Untersuchungspeptid 20, 24 und vorzugsweise ein Ligandentauscher/Ligandenfreisetzer 21 zu dem Multimer 19, das den Helfer-Liganden 13 enthält, zugegeben. Dadurch wird eine Austauschreaktion 22, 25 eingeleitet, an deren Ende Multimere 23, 26 erhalten werden, die mit dem jeweiligen Untersuchungspeptid 20, 24 versehen sind.

Durch dieses Verfahren können Klasse-I-Multimere zunächst ohne Untersuchungspeptid hergestellt und vertrieben werden. Wenn die Multimere dann verwendet werden sollen, kann das Untersuchungspeptid direkt zugegeben werden, vorzugsweise zusammen mit dem Ligandentauscher/Ligandenfreisetzer, und man erhält ein gebrauchsfertiges Klasse-I-Multimer-Reagenz mit entsprechendem Untersuchungspeptid.

Die Erfindung erstreckt sich beispielsweise auf die MHC-Klasse-I-Allotypen, die in den menschlichen Genloci für HLA-A, HLA-B und HLA-C kodiert sind. Sie erstreckt sich auch auf die MHC-Klasse-I-ähnlichen Proteine, die im menschlichen MHC kodiert sind und ebenfalls Peptide binden, d.h. HLA-E und HLA-G. Fernerhin erstreckt sich die Erfindung auf die MHC-Klasse-I-Allotypen, die in den murinen Genloci für H-2K, H-2D und H-2L kodiert sind, sowie auf die Klasse-I-ähnlichen Proteine, die im murinen MHC kodiert sind und ebenfalls Peptide binden, d.h. Qa-1 und Qa-2.

Folgende Anwendungen sind beispielsweise mit der Erfindung realisierbar: Detektion von spezifischen T-Zellen und ihren Frequenzen mit Hilfe von Untersuchungsreagenzien; Isolierung von CTL bestimmter Spezifitäten mit Hilfe von Untersuchungsreagenzien; Aktivierung von CTL bestimmter Spezifitäten mit Hilfe von Untersuchungsreagenzien. Untersuchungsreagenzien sind hierbei insbesondere MHC-Klasse-I-Proteine, Multimere, Partikel und Membranpräparationen, die MHC-Klasse-I-Proteine enthalten, die Untersuchungspeptide enthalten, die mit Hilfe des erfindungsgemäßen Verfahrens vorzugsweise unter Einsatz des Ligandentauschers/Ligandenfreisetzers gegen Helfer-Liganden ausgetauscht worden sind.

### Beispiel 1

Im Folgenden findet sich eine allgemeine Anweisung zur Herstellung eines MHC-Klasse-I-Tetramers mit Hilfe des erfindungsgemäßen Verfahrens.

Klonierung. Zur Einbringung der Gensequenz eines Klasse-I-Proteins in einen Expressionsvektor wird eine Polymerase-Kettenreaktion (PCR) durchgeführt. Der Vorwärtsprimer hat die Sequenz 5' x-y 3', wobei x die Schnittsequenz eines Restriktionsenzyms und y den Beginn des Genes des Klasse-I-Proteins (20 Basenpaare) darstellt. Der Rückwärtsprimer hat die Sequenz 5' e-f 3', wobei e die Schnittsequenz eines Restriktionsenzyms und f eine Sequenz darstellt, die komplementär zum Ende des Gens des Klasse-I-Proteins ist und auch das Stopp-Codon beinhalten kann.

Das PCR-Produkt wird in einen Expressionsvektor kloniert, der einen T7-Promoter enthält, insbesondere durch Zuschneiden der Enden des PCR-Produkts mit Restriktionsenzymen und Zuschneiden der Enden des Expressionsvektors mit Restriktionsenzymen, die zu den Enden des PCR-Produkts passende Überhangsequenzen erzeugen, und anschließende Ligation des PCR-Produkts in den Expressionsvektor mit Hilfe des Enzyms Ligase; oder durch Klonierung des PCR-Produkts in einen Expressionsvektor mit Hilfe eines handelsüblichen PCR-Klonierungskits, insbesondere einen TOPO TA Kit der Firma Invitrogen, oder einen CloneJet Kit der Firma Fermentas. Die Sequenz des Produkts wird dann durch Sequenzieren verifiziert.

### Herstellung des Expressionsstamms.

Das Expressionsplasmid wird in einen E.-coli-Expressionsstamm (z.B. BL21DE3(pLysS)) transformiert, insbesondere durch Herstellung kompetenter Zellen durch Behandlung von E.-coli-Zellen mit Dimethylsulfoxid oder mit Calciumchlorid, Inkubation der behandelten Zellen mit dem Expressionsplasmid, und Selektion der so transformierten Zellen auf Agarplatten, denen das Antibiotikum beigegeben ist, das durch das Enzym, das durch das auf dem Expressionsplasmid vorhandene Resistenzgen kodiert ist, abgebaut wird.

Der Expressionsstamm wird als Flüssigkultur mit 20% Glycerol eingefroren und ist bei -80 °C für mehrere Jahre lagerbar.

### Produktion des MHC-Klasse-I-Proteins.

20 ml LB-Medium mit entsprechendem Antibiotikum (z.B. 100 µg/ml Ampicillin) werden mit 100 µl einer Vorkultur eines Expressionsstamms von Escherichia coli, der ein Plasmid mit einem MHC-Klasse-I-Gen trägt, inokuliert und bei 37 °C in einem Schüttelinkubator über Nacht bis zur stationären Phase angezogen.

Die Kultur wird zentrifugiert (3000 x g, 10 Minuten, 25 °C), der Überstand verworfen, und das Sediment (Zellen) zur Inokulation einer 1-Liter-Kultur desselben Mediums verwendet. Die Kultur wird in einem Schüttelinkubator bei 37 °C bis zu einer Extinktion von 0,4-0,5 bei 600 nm Wellenlänge und 1 cm Küvettendurchmesser, die mit einem Spektrophotometer (ThermoSpectronic Genesys 10UV) gemessen wird, gezogen.

Die Kultur wird mit Isopropyl-β-D-1-thiogalactopyranosid (IPTG) zu einer finalen Konzentration von 0.5 mM versetzt und dann unter den gleichen Bedingungen bis zu einer Extinktion von 1,0 bei 600 nm Wellenlänge und 1 cm Küvettendurchmesser, die mit einem Spektrophotometer (ThermoSpectronic Genesys 10UV) gemessen wird, gezogen.

Die Kultur wird zentrifugiert (13000 x g, 10 Minuten, 25 °C), der Überstand verworfen, und das Sediment in 20 ml Saccharoselösung resuspendiert (25 Gew.-% Saccharose, 1 mM Ethylendiamin¬tetra¬essig¬säure (EDTA), 1 mM Phenylmethyl¬sulfonyl¬fluorid (PMSF), 10 mM Dithiothreitol (DTT), 10 mM Tris(hydroxymethyl)aminomethan (Tris), pH 8.0). Dis Suspension wird in ein 50 ml-Zentrifugenröhrchen transferiert und im Gefrierschrank eingefroren.

### Zell-Lyse

Das Zentrifugenröhrchen mit der Zellsuspension wird im 30 °C-Wasserbad unter Schütteln aufgetaut. Nach dem Auftauen wird auf Eis oder im Kühlraum weitergearbeitet.

Fragmentierung der DNA: Die Suspension wird mit der Sonde eines Ultraschallgeräts beschallt, solange bis sie nach Anschauung nicht mehr viskos ist.

Die Suspension wird zentrifugiert (40000 x g, 15 Minuten, 4 °C), der Überstand verworfen, und das Sediment durch Beschallung in 20 ml Detergens-Puffer resuspendiert (25 Gew.-% Saccharose, 1 Vol.-% Triton X-100, 5 mM EDTA, 2 mM DTT, 50 mM Tris, pH 8.0).

Die Suspension wird zentrifugiert (40000 x g, 15 Minuten, 4 °C), der Überstand verworfen, und das Sediment durch Beschallung in 20 ml Harnstoffpuffer resuspendiert (2 M NaCl, 2 M Harnstoff, 2 mM DTT, 25 mM Tris pH 8.4).

Die Suspension wird zentrifugiert (40000 x g, 15 Minuten, 4 °C), der Überstand verworfen, und das Sediment durch Beschallung in 20 ml TBS resuspendiert (150 mM NaCl, 0.5 mM PMSF, 20 mM Tris pH 7.5).

Die Suspension wird zentrifugiert (40000 x g, 15 Minuten, 4 °C), der Überstand verworfen, und das Sediment durch Beschallung in 10 ml Denaturierungslösung resuspendiert (8 M Harnstoff, 50 mM 4-(2-hydroxyethyl)-1-piperazin-ethansulfonsäure (HEPES), 100 µM β-mercaptoethanol, pH 6.5), und die Suspension für 48 Stunden bei 4 °C unter leichtem Schütteln inkubiert.

Die Suspension wird zentrifugiert (40000 x g, 15 Minuten, 4 °C). Das Sediment wird verworfen und die Zentrifugation wiederholt. Das Sediment wird wiederum verworfen und die klare Lösung durch einen 0,22-µm-Filter filtriert, aliquotiert und bei -20 °C bis zur weiteren Verwendung aufbewahrt. Die Proteinkonzentration wird durch Messung der Extinktion bei 280 nm gegen Wasser bestimmt. Der Extinktionskoeffizient ε eines Klasse-I-Proteins unter diesen Bedingungen beträgt etwa 85000 M-1cm-1.

Auf die hier beschriebene Weise werden entweder die schwere Untereinheit von MHC Klasse I und die leichte Untereinheit (beta-2 microglobulin, β₂m) getrennt in zwei verschiedenen Ansätzen hergestellt (um später bei der Faltung gemischt zu werden), oder ein Fusionsprotein aus der schweren und der leichten Kette wird entsprechend als einziges Protein hergestellt.

### Faltung des Klasse-I-Proteins.

Ein Liter Faltungspuffer (100 mM Tris-Cl pH 8,0, 0,5 M Arginin, 2 mM EDTA, 0,5 mM oxidiertes Glutathion, 5 mM reduziertes Glutathion) wird unter Rühren bei 4 °C tropfenweise mit entweder 10 mg denaturiertem β2m und 30 mg denaturierter schwerer Untereinheit oder mit 20 mg eines Fusionsproteins aus der schweren und der leichten Kette in Denaturierungslösung versetzt. Ein Helfer-Ligand wird ebenfalls hinzugegeben, typischerweise in einer Konzentration zwischen 100 nM und 1 mM. Die Lösung wird 12 Stunden weiter gerührt.

Die Lösung wird in einem Druckfiltrationsgerät auf etwa 100 ml und dann in einem zentrifugalen Konzentrationsgerät (Trenngrenze: 30000 Da) auf etwa 1 ml konzentriert und dann zentrifugiert (16000 x g, 4 °C, 15 min); das Sediment wird verworfen.

Der Überstand wird filtriert (0,22 µm) und auf eine in TBS (150 mM NaCl, 25 mM Tris-Cl pH 7,5) äquilibrierte Gelfiltrationssäule (z.B. GE Healthcare HiLoad 16/60 Superdex 75 prep grade) aufgetragen. Der Peak des gefalteten Klasse-I-Proteins wird durch Analyse der Proben mit SDS-Polyacrylamid-Gelelektrophorese identifiziert: er enthält sowohl schwere als auch leichte Kette und weist ein apparentes Molekulargewicht von etwa 60000 Da auf.

Die entsprechenden Fraktionen werden vereinigt. Die Proteinkonzentration wird wie oben bestimmt.

### Herstellung eines Klasse-I-Tetramers.

Gefaltete MHC-Klasse-I-Proteine werden in einer Konzentration von 5 µM in Biotinylierungspuffer (50 mM Tris-Cl pH 7,4; 150 mM NaCl; 1 mM Biotin; 5 mM Adenosintriphosphat; 5 mM MgCl₂) aufgenommen und mit 0,1 µM rekombinanten BirA-Biotinylierungsenzym versetzt. Die Reaktion wird für 12 Stunden bei 25 °C inkubiert.

Die Tetramerisierung wird durch Zugabe von 20 µM fluoreszenzmarkiertem Avidin oder Streptavidin (z.B. phycoerythrin-UltraAvidin, Leinco) induziert, und die Reaktion wird für 15 Minuten bei 4 °C inkubiert.

Die Tetramer-Komplexe werden durch Gelfiltration mit einer Superdex S-200 Säule (in TBS (25 mM Tris-Cl pH 7.4; 150 mM NaCl) äquilibriert) gereinigt.

Die entsprechenden Fraktionen werden vereinigt. Die Proteinkonzentration wird wie oben bestimmt.

### Zugabe des Untersuchungspeptids.

Um ein peptidgebundenes Tetramer zu erhalten, wird eine Lösung des Tetramers in TBS mit 10 µM des entsprechenden Untersuchungspeptids und mit 10 mM Ligandentauscher/Ligandenfreisetzer (oder der jeweils geeigneten Konzentration, abhängig von der chemischen Natur des Ligandentauschers/Ligandenfreisetzers) versetzt. Die Reaktion wird bei 25 °C für 15 Minuten inkubiert.

Falls erforderlich, werden die Peptid-Tetramer-Komplexe durch Gelfiltration mit einer Superdex S-200 Säule (in TBS äquilibriert) gereinigt. Die entsprechenden Fraktionen werden vereinigt. Die Proteinkonzentration wird wie oben bestimmt.

### Reaktion des Tetramers mit T-Zellen und Durchflusszytometrie.

200 000 isolierte T-Zellen (die Zahl wird mit Hilfe einer Neugebauer-Zählkammer unter dem Mikroskop bestimmt) werden in 1 ml FACS-Puffer (TBS plus 2% fötalem Kälberserum und 5 mM NaN₃) mit gereinigtem Peptid-Tetramer-Komplex zu einer finalen Konzentration von 0.5 mg/ml versetzt. Die Reaktion wird bei 4 °C für eine Stunde inkubiert.

Die Zellen werden zentrifugiert (800 x g, 5 Minuten, 4 °C) und der Überstand entfernt. Die Zellen werden zweimal mit FACS-Puffer gewaschen und dann in Fixierungspuffer (PBS, 2% Paraformaldehyd) resuspendiert.

Durchflusszytometrie erfolgt mit einem handelsüblichen Gerät (z.B. BectonDickinson FACSAria oder Partec CyFlow Space).

### Beispiel 2

Im Folgenden wird die Verwendung des Kits beschrieben. Ein Kit, der ein Vorlösungsuntersuchungsreagens enthält, wird bereitgestellt.

Um ein Untersuchungsreagens zu erhalten, wird die Vorlösung des Vorlösungsuntersuchungsreagens mit 10 µM des entsprechenden Untersuchungspeptids und 10 µM Ligandentauscher/Ligandenfreisetzer (oder der jeweils geeigneten Konzentration, abhängig von der chemischen Natur des Ligandentauschers/Ligandenfreisetzers) versetzt. Die Reaktion wird bei 25 °C für 15 Minuten inkubiert.

Falls erforderlich, wird das Untersuchungsreagens durch Gelfiltration mit einer Superdex S-200 Säule (in TBS äquilibriert) gereinigt. Die entsprechenden Fraktionen werden vereinigt. Die Proteinkonzentration wird wie oben bestimmt.

Die Reaktion des Untersuchungsreagens mit T-Zellen und Detektion der Komplexe erfolgt wie zuvor beschrieben.

### Beispiel 3

In Folgenden wird das erfindungsgemäße Verfahren anhand eines konkreten Ausführungsbeispiels näher beschrieben.

Zunächst wird das murine MHC-Klasse-I-Protein H-2K^{b} nach dem oben beschriebenen Verfahren in *E*.*-coli*-Zellen hergestellt und mit einem Biotinmolekül versehen. Daraufhin wird es mit dem ebenfalls in *E*.-*coli*-Zellen hergestellten humanen Beta-2-Mikroglobulin und mit dem Helfer-Liganden Phenylalanylalanyl-prolyl-glycyl-asparaginyl-tyrosyl-prolyl-alanyl-leucin (SEQ ID NO: 2), also einem Peptid der Sequenz FAPGNYPAL (die Buchstaben der Peptidsequenz entsprechen dem Einbuchstabencode für Aminosäuren nach den Bestimmungen der International Union for Pure and Applied Chemistry) nach dem oben beschriebenen Verfahren gefaltet. Daraufhin wird der Helfer-Ligand FAPGNYPAL mit Hilfe des Ligandentauschers/Ligandenfreisetzers Glycyl-Leucin also einem Peptid der Sequenz GL, gegen das Untersuchungspeptid Seryl-isoleucyl-isoleucyl-asparaginylphenylalanyl-glutamyl-lysyl-leucin (SEQ ID NO: 3), also einem Peptid der Sequenz SIINFEKL, nach dem oben beschriebenen Verfahren ausgetauscht. Daraufhin wird der Komplex nach dem oben beschriebenen Verfahren mit Streptavidin umgesetzt, das mit dem Fluoreszenzfarbstoff Fluoreszein chemisch verbunden ist und anschließend gereinigt. Dadurch ist ein Untersuchungsreagens hergestellt, welches anschließend verdünnt und mit dem T-Zell-Klon B3Z umgesetzt wird, der einen T-Zell-Rezeptor trägt, der an den Komplex aus H-2K^{b} und SIINFEKL bindet. Daraufhin werden die so gefärbten Zellen in einem Durchflusszytometer analysiert.

### Bezugszeichenliste:

- 1:: Entfaltetes MHC-Klasse-I-Protein
- 2:: Untersuchungspeptid
- 3:: Prozedur der Faltung des MHC-Klasse-I-Proteins mit dem Untersuchungspeptid
- 4:: MHC-Klasse-I-Protein gefaltet mit gebundenem Untersuchungspeptid
- 5:: Vorstufe des Ankerelements, hier: Reagenz zur Biotinylierung
- 6:: Prozedur der Biotinylierung
- 7:: MHC-Klasse-I-Protein gefaltet mit gebundenem Untersuchungspeptid und biotinyliert
- 8:: Multimerisierungselement, hier: Streptavidin
- 9:: Prozedur der Bindung von (7) an (8)
- 10:: Multimer, hier: Tetramer mit Untersuchungspeptid (2)
- 11:: Untersuchungspeptid (chemisch verschieden von (2))
- 12:: Multimer, hier: Tetramer mit Untersuchungspeptid (11)
- 13:: Helfer-Ligand
- 14:: Prozedur der Faltung des MHC-Klasse-I-Proteins mit dem Helfer-Ligand
- 15:: MHC-Klasse-I-Protein gefaltet mit gebundenem HelferLigand
- 16:: Prozedur der Biotinylierung
- 17:: MHC-Klasse-I-Protein gefaltet mit gebundenem HelferLigand und Ankerelement (z.B. Biotin)
- 18:: Prozedur der Bindung von (17) an Streptavidin
- 19:: Multimer mit MHC-Klasse-I-Proteinen mit gebundenem Helfer-Ligand
- 20:: Untersuchungspeptid
- 21:: Ligandentauscher/Ligandenfreisetzer
- 22:: Prozedur des Austauschs des Helfer-Liganden durch den Ligandentauscher/Ligandenfreisetzer gegen das Untersuchungspeptid (20)
- 23:: Multimer, hier: Tetramer mit Untersuchungspeptid (20)
- 24:: Untersuchungspeptid (chemisch verschieden von (20))
- 25:: Prozedur des Austauschs des Helfer-Liganden durch den Ligandentauscher/Ligandenfreisetzer gegen das Untersuchungspeptid (24)
- 26:: Multimer, hier: Tetramer mit Untersuchungspeptid (24)

## Patentansprüche

1. Verfahren zum Herstellen eines Untersuchungsreagens, wobei
a) einem ungefalteten MHC-Klasse-I-Protein in einer Ausgangslösung ein gegen ein Untersuchungspeptid austauschbarer Helfer-Ligand zugefügt wird, so dass eine Vorlösung des Untersuchungsreagens mit einem gefalteten MHC-Klasse-I-Protein vorliegt, das den Helfer-Liganden gebunden aufweist, und
b) ein Ligandentauscher/Ligandenfreisetzer zu dem gefalteten MHC-Klasse-I-Protein mit dem daran gebundenen Helfer-Liganden gegeben wird, wobei der Ligandentauscher/Ligandenfreisetzer eine geringere Affinität zum MHC-Klasse-I-Protein aufweist als der Helfer-Ligand und b1) eine Aminosäure, b2) eine modifizierte Aminosäure oder b3) ein Peptid aus 2 oder 3 Aminosäuren ist.

2. Verfahren nach Anspruch 1, wobei der Helfer-Ligand eine geringere Affinität zum MHC-Klasse-I-Protein aufweist als das Untersuchungspeptid und/oder wobei der Helfer-Ligand ein Peptid aus 2, 3, 4, 5, 6, 7, 8, 9, 10, oder 11, bevorzugt 8, 9, 10 oder 11, besonders bevorzugt 9 oder 10 Aminosäuren ist.

3. Verfahren nach einem der vorhergehenden Ansprüche, wobei ein Helfer-Ligand verwendet wird, der ohne vorherige Spaltung gegen das Untersuchungspeptid ausgetauscht werden kann.

4. Verfahren nach einem der vorherigen Ansprüche, wobei mehrere gefaltete MHC-Klasse-I-Proteine zu einem Multimer zusammengefügt werden und wenigstens eines der gefalteten MHC-Klasse-I-Proteine den Helfer-Liganden aufweist.

5. Verfahren zum Erhalt eines Analyseergebnisses, umfassend den Schritt, dass ein Untersuchungspeptid und ein nach einem Verfahren nach einem der Ansprüche 1-4 hergestelltes Untersuchungsreagens zusammen gegeben werden, so dass sich eine Analyselösung ergibt, unter Bedingungen, unter denen der Helfer-Ligand ohne seine vorherige Spaltung durch das Untersuchungspeptid ersetzt wird.

6. Verfahren nach Anspruch 5, wobei die Analyselösung mit einer Zellprobe zusammengeführt wird, sodass das Untersuchungsreagens mit einem spezifischen Element der Zellprobe komplexiert, so dass ein Komplex vorliegt.

7. Verfahren nach Anspruch 6, wobei nach dem Zusammenführen ein Detektieren eines Signalgebers, der vorzugsweise ein Fluoreszenzfarbstoff ist, erfolgt, insbesondere durch eine Durchflusszytometrie.

8. Verfahren nach einem der Ansprüche 5 bis 7, wobei die Zellprobe CTL umfasst und die Bildung des Komplexes aus Zellprobe und Untersuchungsreagens dazu führt, dass bestimmte CTL aktiviert werden, oder dazu führt dass bestimmte CTL aus der Gesamtmenge abgetrennt werden, und wobei das Abtrennen insbesondere mittels durchflusszytometrischer oder chromatographischer Verfahren erfolgt, sodass ein Trennergebnis und ein Rest vorliegen.

## Claims

1. A method for producing an examination reagent, wherein
a) a helper ligand, which can be exchanged with an examination peptide, is added to an unfolded MHC-class-I-protein in an initial solution, so that a pre-solution of the examination reagent with a folded MHC-class-I-protein is provided, which features the bonded helper ligand, and
b) a ligand exchanger/ligand releaser is added to the folded MHC-class-I-protein featuring the bonded helper ligand, wherein the ligand exchanger/ligand releaser has a lesser affinity with the MHC-class-I-protein than the helper ligand and b1) is an amino acid, b2) is a modified amino acid or b3) is a peptide of 2 or 3 amino acids.

2. The method according to claim 1, wherein the helper ligand has a lesser affinity with the MHC-class-I-protein than the examination peptide and/or wherein the helper ligand is a peptide of 2, 3, 4, 5, 6, 7, 8, 9, 10, or 11, preferably of 8, 9, 10 or 11, most preferably of 9 or 10 amino acids.

3. The method according to one of the afore-mentioned claims, wherein a helper ligand is used, which can be exchanged with the examination peptide without prior cleavage.

4. The method according to one of the afore-mentioned claims, wherein several folded MHC-class-I-proteins are assembled to form a multimer and at least one of the folded MHC-class-I-proteins features the helper ligand.

5. A method for obtaining an analysis result, comprising the step of mixing an examination peptide and an examination reagent produced with the method according to one of the claims 1-4, so as to provide an analysis solution under conditions in which the helper ligand is replaced with the examination peptide without prior cleavage.

6. The method according to claim 5, wherein the analysis solution is mixed with a cell sample, so that the examination reagent complexes with a specific element of the cell sample, so that a complex is provided.

7. The method according to claim 6, wherein a marker, which is preferably a fluorescent dye, is detected after mixing, in particular by flow cytometry.

8. The method according to one of the claims 5 to 7, wherein the cell sample comprises CTLs and the formation of the complex composed of the cell sample and the examination reagent leads to the activation of specific CTLs or to the separation of specific CTLs from the total amount, and wherein the separation is carried out more specifically by means of flow cytometry or chromatographic methods, so that a separation result and a rest are provided.

## Revendications

1. Procédé de préparation d'un réactif de recherche, où
a) un ligand auxiliaire remplaçable par un peptide de recherche est ajouté à une protéine CMH de classe I non pliée dans une solution de départ, de sorte que l'on obtient une pré-solution du réactif de recherche avec une protéine CMH de classe I repliée qui comporte le ligand auxiliaire lié, et
b) un échangeur de ligand/libérateur de ligand est ajouté à la protéine CMH de classe I repliée à laquelle est lié le ligand auxiliaire, où l'échangeur de ligand/libérateur de ligand a une moindre affinité avec la protéine CMH de classe I que le ligand auxiliaire et b1) est un acide aminé, b2) est un acide aminé modifié ou b3) est un peptide composé de 2 ou 3 acides aminés.

2. Procédé selon la revendication 1, où le ligand auxiliaire a une moindre affinité avec la protéine CMH de classe I que le peptide de recherche et/ou où le ligand auxiliaire est un peptide composé de 2, 3, 4, 5, 6, 7, 8, 9, 10 ou 11, préférablement de 8, 9, 10 ou 11, plus préférablement de 9 ou 10 acides aminés.

3. Procédé selon l'une quelconque des revendications précédentes, où l'on utilise un ligand auxiliaire qui peut être échangé contre le peptide de recherche sans clivage préalable.

4. Procédé selon l'une quelconque des revendications précédentes, où l'on assemble plusieurs protéines CMH de classe I repliées en un multimère et au moins une des protéines CMH de classe I repliées présente le ligand auxiliaire.

5. Procédé d'obtention d'un résultat d'analyse, comprenant l'étape consistant à mélanger un peptide de recherche et un réactif de recherche préparé selon un procédé selon l'une des revendications 1 - 4, de sorte que l'on obtient une solution d'analyse dans des conditions dans lesquelles le ligand auxiliaire peut être remplacé par le peptide de recherche sans clivage préalable.

6. Procédé selon la revendication 5, où la solution d'analyse est mélangée avec un échantillon de cellule, de sorte que le réactif de recherche se complexe avec un élément spécifique de l'échantillon de cellule de manière, de sorte que l'on obtient un complexe.

7. Procédé selon la revendication 6, où après le mélange, l'on détecte un marqueur, de préférence un colorant fluorescent, en particulier par cytométrie en flux.

8. Procédé selon l'une quelconque des revendications 5 à 7, où l'échantillon de cellules comprend des CTL et la formation du complexe de l'échantillon de cellules et du réactif de recherche conduit à l'activation de CTL spécifiques ou à la séparation de CTL spécifiques de l'ensemble, et où la séparation s'effectue en particulier par des procédés de cytométrie en flux ou chromatographiques, de sorte que l'on obtient un résultat de la séparation et un reste.
